# EUROPEAN PATENT APPLICATION

(11) **EP 4 670 947 A1**
(43) Date of publication of application: **31.12.2025**
(21) Application number: 24306001.9
(22) Date of filing: 24.06.2024
(51) Int. Cl.: B29C 45/00, A44B 18/00, A61F 13/62, B29C 37/00, B29C 45/44, F16B 5/07, B29C 71/02, B29L 31/00

(54) **FASTENING ELEMENT AND ITS PRODUCTION**

(71) Applicant: Aplix, 44850 Le Cellier (FR)
(72) Inventor: CRUZ, Rafael, 44850 LE CELLIER (FR); RAYMOND, Lucile Sophie, 44850 LE CELLIER (FR)
(74) Representative: Cabinet Beau de Loménie

(57) **Abstract**

A method of producing a fastening element having a surface and retaining elements projecting from the surface comprises injection-molding into cavities of at least a part of a mold and imparting conversion to structures obtained in said cavities from said injection-molding into deformed structures by demolding. Annealing reverses an extent of said conversion by application of energy to at least parts of the deformed structures in a released state of being exposed to a fluid.

## Description

### Technical field

The invention relates to a fastening element having a surface and retaining elements projecting from the surface and a method for at least a portion of the fastening element.

### State of the art

Hook and loop fastening systems are commonly known, e.g. as a pair panels, one with hooks projecting from a surface and one with complementary loops projecting from a surface, which panels may be fastened or fixed to each other via interaction between the hooks and loops. An alternative to such paring of a panel with hooks with a panel with loops is the use of panels with projections having shapes of mushrooms or hooks, in particular pairs of hooks, so that a pair of panels provides a pair of projecting structures interacting with each other so that the panels may be fastened or fixed to each other. Injection-molding thermoplastic resins is a technique available for producing the panels and/or projecting structures like hooks and/or the loops on the panels. A suitable mold is shown in US 2009/0106955 A1. US 2015/0313320 A1 shows an adaptation of injection-molding to the use of amorphous resins in injection-molding.

A method and a mold for forming the panel with hooks are shown in US 6,224,364 B1. FR 3 093 671 shows molding hook-shaped or mushroom-shaped projections onto a base to obtain a panel for a fastening system. A single injection-molding operation forming a single piece panel for a fastening system with hooks is shown in US 2011/0111165 A1.

Ends of projecting structures of an intermediate molded product are deformed by application of heat according to WO 2022/268465 A1 to convert the intermediate molded product into a fastening element with a fastening function of its projecting structures.

For avoiding deformation of injection-molded projections in a demolding operation, WO 2005/096864 A1 shows a one piece molding of a fastening element with projecting hooks from specific resins.

### Presentation of the invention

The invention aims at improving versatility, efficiency and/or innovation in providing fastening concepts and products or related assembling concepts. The invention aims at allowing a new material choice for a fastening element with excellent efficiency and/or performance, being excellent in engaging force with the hook-and-loop fastener loops member, such as having excellent shearing stress and/or peeling strength, notably for multiple cooperation cycles with loop counterparts, and at least partially available via injection-molding. Additionally, with such method, it could be possible to make functional retaining elements in various raw materials that was not possible by the past due to uncompromising methods of the art requesting strict parameters for obtaining functional hooks just after demolding the injected products. Additionally, the injection-molding domain being a discontinuous process, it is difficult to apply a "post treatment" for such discontinuous manufactured products, especially on discrete area comprising hooks or deformed hooks. Furthermore, the injection-molding domain being a discontinuous process, for multiple and/or small discrete areas of hooks it is much more difficult to apply an adapted "post treatment".

The invention provides a method of producing at least a portion of a fastening element having a surface and retaining elements projecting from the surface, comprising injection-molding into cavities of at least a part of a mold and imparting conversion to structures, obtained in said cavities from said injection-molding, into deformed structures by demolding. The method of the invention includes annealing for reversing an extent of said conversion by application of energy to at least parts of the deformed structures (in particular, at least a part of the outside surface of the deformed structures, in particular at least a part of the outside surface of the free ends of the deformed structures) in a released state of being exposed to a fluid, e.g. an atmosphere, in particular, fully exposed to a fluid.

The atmosphere is preferably non-pressurized and can be a normal or natural atmosphere without any specific measures to establish an atmosphere. The energy is preferably a radiation energy such as heat radiation energy and/or infrared energy and/or microwave energy. In particular, the energy is applied without additional pressure and/or without additional mechanical contact and/or by impinging a heated fluid such as hot air and/or hot water and/or other hot fluid. A further option is using fluid bath comprising hot beads. In some preferred cases, the energy is applied without any calendering step, the calendering step comprising at least one roll.

Thus, annealing can simply be a one-step operation of heating and/or infrared irradiating an intermediate product or at least its projections or a part of them, in particular an upper end part distant to the surface. In alternative, in some cases, annealing can be multiple step operations of heating and/or infrared irradiating an intermediate product or at least its projections or a part of them, in particular an upper end part distant to the surface. With such multiple step operations, annealed zones are not too overheated, namely the undesired zones. The multiple-step operations are a means to improve controlling a progressive conduction effect in the material. Using annealing particularly allows obtaining different dimensions of the projections per annealed zones.

The conversion and related deformation result from the use of an amorphous resin in said injection-molding into cavities. On average, the (post-treated) retaining elements may project from the surface to a height which is less than 90% of a height of projections from the surface of the (deformed) structures formed by imparting said conversion. In particular exclusively an amorphous resin may be used here or said injection-molding into cavities may be performed with amorphous and/or non-amorphous resins (that could be a crystalline resin and/or semi-crystalline resin) to provide in each of the cavities a volume ratio of amorphous zones of resin and non-amorphous zones of resin of more than 1. It is possible that each of the structures obtained in the respective one of the cavities being composed of a resin comprising amorphous zones and non-amorphous zones, the amorphous zones represent more than 50% of the volume of the cavity, notably more than 60%, in particular more than 70% of, preferably more than 80%, more preferably more than 95%, the remaining volume of the cavity being non-amorphous zones. According to an option, each of the structures obtained in the respective one of the cavities being composed of a resin comprising amorphous zones and non-amorphous zones, the amorphous zones represent less than 99% of the volume of the cavity, the remaining volume of the cavity being non-amorphous zones. The effect of the present invention can be more expressed when filling is in these ranges due to the high level of deformation strain (or deformation stress) applied to the structures during demolding. The conversion and related deformation can also result from the use of a crystalline thermoplastic resin and / or semi crystalline thermoplastic resin in said injection-molding into cavities for which the effect of the present invention can be expressed. Indeed, by using the method according to the invention using crystalline thermoplastic resin and / or semi crystalline thermoplastic resin, the obtained hooks are not only "good" (i.e. having good gripping properties, such as shear and/or peel and/or equivalent) but become "excellent" (i.e. having excellent gripping properties, such as shear and/or peel and/or equivalent) and/or show a hook shape that is not possible to obtain using only injection-molding alone.

Also, a fastening element is provided by the invention and is defined in the claims. The fastening element may be one having a base surface and injection-molded retaining elements, preferably one obtainable by the method of the invention. The fastening element may be one having a base surface and injection-molded retaining elements at least partially made of an amorphous resin, preferably one obtainable by the method of the invention. Retaining elements of the fastening element may be injection-molded onto said surface or may be part of a single-piece fastening element. Retaining elements of the fastening element may be integral with said surface of the base and/or may be integral with the base and/or may be integral with the support.

Generally, the invention provides a fastening element having a base surface and injection-molded retaining elements at least partially made of an amorphous resin and/or crystalline resin and projecting from the base surface up to and with a retention portion, wherein a further surface being a surface of each of the retaining elements and visible in a view showing the retention portion and onto a plane, on which the retaining element extends between the retention portion and the base surface, has an area comprising a profiled area, wherein, when subjected to an analysis according to the description of the retaining elements, said profiled area shows, when measuring in accordance with ISO 25178-2:2021, at least one of
a) spatial isotropy or directionality of a texture with a value of Str above 0.20 and/or up to 1.0,
b) symmetry with regard to a mean line of a roughness profile with a value of Ssk above 2.3 or below -1, and
c) sharpness of a roughness profile with a value of Sku above 5 or below 2.

The view may be a view onto a straight or a curved plane. The fastening element of the invention may be one obtained by the method of the invention.

In particular the invention allows the molding of hooks directly into brackets with certain amorphous materials such as PC/ABS. This eliminates the need for a separate fastener and the labor to insert the fasteners onto a bracket or the risk of incorrect positioning thereof. It also prevents a potential for rattles and also allows establishing small gap heights for hook and loop systems that could be a source of noise. The need to reduce the sound is more and more a current and important matter in view of the noise reduction made in the recent years, for example vehicle noise reduction, especially for a hybrid and/or electric vehicle.

Uses of the fastening element of the invention are uses in industry, uses for fastening systems as mentioned initially in this description, uses for assembling or fixing vehicle (i.e. automobile) parts in particular in a cabin or interior compartment of an automobile like a passenger compartment, uses for diapers or packages as a closure. In particular, in automotive for overhead systems like sunroof/moonroof systems in a cabin or interior compartment of an automobile ABS or PC/ABS resin can be an excellent and desired material choice. In particular, in this context the invention avoids an extent of straightness of injection-molded projections by annealing such straight or deformed intermediate structures/intermediate hooks to return a shape and strength of the hook of an intended state. An entire bracket or hook as a projection from a surface can be formed easily with the invention's method. The uses of the fastening element of the invention are in particular uses without numerous cycles of opening and closing so that a shape memory of projections of a fastening element has no or low relevance. Then the fastening element of the invention can be used for limited openings and closings like in a use for an automotive overhead system or applications that require one-time or a limited number of opening and/or closing operations.

Preferably, the injection-molding in the invention's method is a one (single) and not a multistage operation for the sake of manufacturing efficiency. In some cases, the injection-molding in the invention's method is a two or three injection steps.

Preferably, the retaining elements and the base surface, notably also the base, can be made together by injection-molding, using notably one, two or three steps. In such case, the retaining elements and the base surface is so-called respectively injection-molded retaining elements, injection-molded base surface and injection-molded base and are generally manufactured using closed mold, such closed mold can comprise air vents.

The fastening elements can comprise retaining elements formed integrally by means of injection-molding step, or in other words, by a discontinuous process by opposition to a continuous process, such as extrusion process which has an opened mold. Thus, the fastening element provided by the invention is integral with the base surface and/or the base without any risk of separation between the base and the retaining elements and/or the fastening element provided by the invention is integral with the base surface and the base and the support without any risk of separation between the support, the base and the retaining elements.

Preferably, the base surface and/or a base as demolded (before annealing step and/or after annealing step) can comprise an injection point(s) and/or injection ejector mark(s) and/or injection parting line(s) and/or injection clearance(s)/draft(s) and/or text(s) in relief.

### Presentation of the drawings

The invention and its advantages will be better understood upon reading the detailed description made hereafter of different aspects of the invention, given by way of non-limiting examples. This description makes reference to the pages of appended drawings, in which:
[Fig. 1] Figure 1 presents a microscopic view onto a portion of a fastening element according to the invention with a hook-shaped injection-molded projection on a base.
[Fig. 2] Figure 2 presents a microscopic view on a pair of hook-shaped injection-molded projections on a base from a crystalline thermoplastic resin.
[Fig. 3A, 3B] Figure 3A presents a pair of protrusions of a thermoplastic amorphous resin structure before and Figure 3B after annealing.
[Fig. 4A, 4B] Figure 4A presents a pair of hook-shaped injection-molded projections of a thermoplastic amorphous resin (produced with the mold used for the structure of Fig. 2) before and Figure 4B after annealing.
[Fig. 4C] Figure 4 C is a superposition of the images of Figure 4A and Figure 4B.
[Fig. 5] Figure 5 presents a pair of protrusion of a thermoplastic crystalline resin structure produced with the mold used for the structure of Figure 3A.
[Fig. 6A, 6B] Figure 6A presents a pair of hook-shaped injection-molded projections of a thermoplastic amorphous resin (and produced with the mold used for the structure of Figure 2 before and Figure 6B after annealing.
[Fig. 7] Figure 7 presents a microscopic view on a pair of hook-shaped injection-molded projections on a base from another crystalline thermoplastic resin (and produced with the mold used for the structure of Fig. 2).

In all the figures, similar elements are designated by identical numerical references.

### Detailed description

### Shape options for the fastening element and related functions

For suitable shapes for the fastening element and/or its retaining elements reference is made to US 2015/0313320 A, US 6,224,364 B1, WO 2005/096864 A1, US 2009/0106955 A1, WO 2014/083245 A1, and US 2011/0111165 A1, which disclose shapes of a fastening element and/or its retaining elements, in particular hooks or stem and head structures like mushroom-shaped projections.

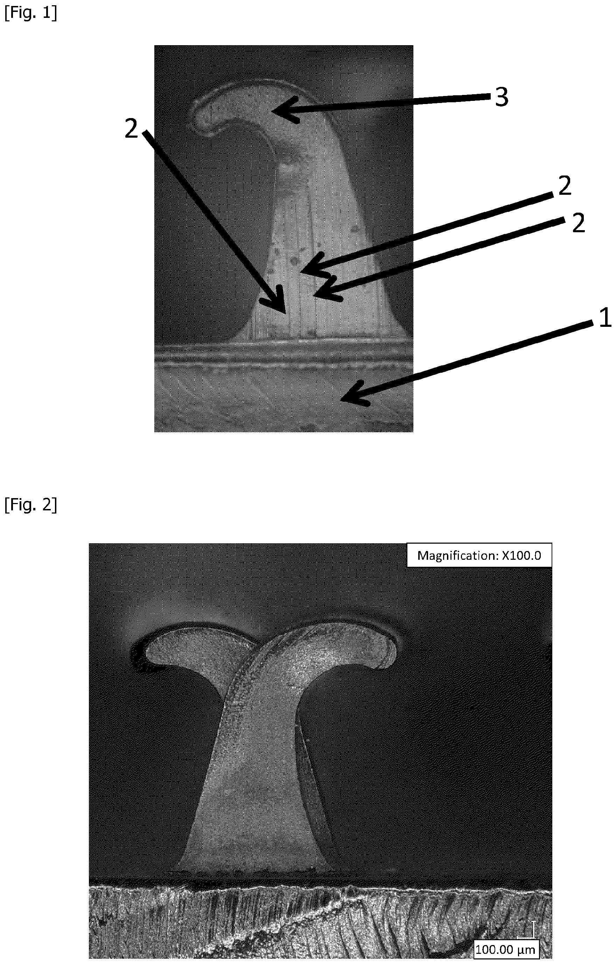

As illustrated in Figure 1, a hook may be formed on a surface of a support or base 1.

The support 1 may comprise at least 2, 3, 4, or 5 and/or less than 70 portions, which are not in a same plane. In a fastening system with a pair of fastening elements one or more fixing means may be arranged in a or the support of a fastening element, in particular the fastening element of the invention, preferably distal from the retaining elements, for directly or indirectly attaching the fastening system e.g. to a chassis of a vehicle. Fixing means or retaining elements of the support may comprise a hole, through hole, rivet, self-thread, a self-tap screw, and or a spring pin, or may be established with ultrasonic welding, insert-molded threaded insert, heat set inserts, tap-in inserts, heat welding, solvent bonding, adhesive bonding, molded snap fit, molded tabs, thermal welding, staking, or a combination thereof. The support is preferably a single piece and may be porous or non-porous and not hollow or hollow for weight reduction purpose. More preferably, the support is a complex support, such as one comprising numerous portions for different fastening means and/or hollow portions. Imparting more conversion to structures allows having high fastening effects, and thus having the retaining elements highly deformed. The effect of the present invention can be expressed easily for the fastening elements independently of the shape of the support and/or the number of portions or other features complexifying the fastening system or fastening element.

The support or base 1 may constitute at least 50%, or at least 70%, or at least 80% or at least 90% or at least 95% of the volume of the fastening element and/or may be defined by two, preferably parallel, surfaces together with at least one side surface linking these preferably parallel surfaces.

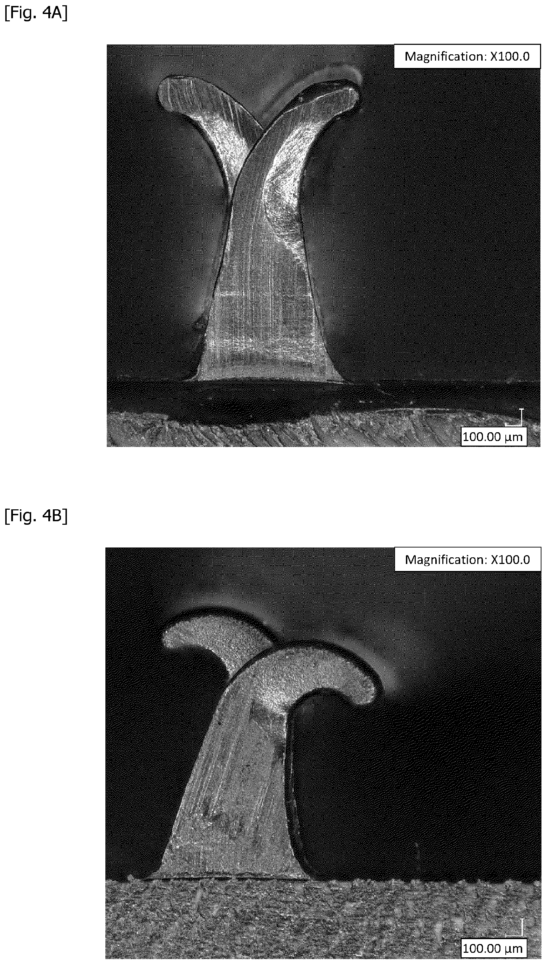

The retaining elements are preferably hooks, in particular with pairs of hooks like dual hooks as illustrated e.g. in Figure 4B. Exemplary sets of specifications for retaining elements, specifically hooks of the fastening element of the invention are shown in Table 1.

**[Table 1]**

| | | | | | | |
|---|---|---|---|---|---|---|
| fastening element type | 1 | 2 | 3 | 4 | 5 | 6 |
| hook height (mm) | 1.42 | 0.91 | 0.55 | 0.4 | 0.3 | 4 |
| hook thickness (mm) | 0.38 | 0.38 | 0.25 | 0.15 | 0.15 | 1.65 on bottom |
| | | | | | | 0.51 on top |
| distance between hooks on the surface on which they are formed (mm) | 0.38 | 0.76 | 0.38 | suitably set | 0.3 | 3.35 |
| distance between ends of hooks parallel to the surface on which they are formed (mm) | 2.5 | 1.52 | 1.88 | 1.4 | 1 | 5 |
| Number of hooks per cm² | 51 | 58 | 113 | 208 | 347 | suitably set |

Around each of the exemplary sets of specifications, suitable specifications for retaining elements are within preferable ranges having lower limits of 20% below the exemplified values and upper limits 20% above the exemplified values, as far as concrete values are indicated above.

The volume of hooks of a fastening element of the invention may be 100 times, preferably 1000 times, more preferably 100 000 and 100 000 000 times smaller than a volume of its support (in line with specifications shown in US 2011/0111165 A1).

The fastening element of the invention may be formed with a density of hooks on the surface of the base between one of lower limits of 20 hooks /cm², in particular 30 hooks /cm² or preferably 70 hooks /cm², and one of upper limits of 400 hooks /cm², in particular 370 hooks /cm² or preferably 150 hooks /cm². According to an option, such density can be measured in a hook area, i.e. in an area comprising hooks regularly spaced and/or arranged according to at least one pattern. The hooks may project from a surface of the support or base 1 up to a height measured perpendicular to the surface of at least 0.2 mm, in particular at least 0.3 mm, preferably at least 0.4 mm, and/or at most 4 mm, in particular at most 3 mm, preferably at most 2 mm. A ratio of a width of the hooks on this surface of the support or base 1 to this height of the hooks may between one of lower limits of 0.1, in particular 0.15, preferably 0.20 and one of upper limits of 0.5, in particular 0.45, preferably 0.40. These specifications preferably apply to average values obtained for the hooks on a surface of the fastening element of the invention. The effect of the present invention can be even more expressed when the retaining elements are in these ranges. The fastening element of the invention could be arranged on the surface in order to define one or more hook areas. The number of hook areas could be more than or equal to 1, in particular more than or equal to 2, in particular more than or equal to 3 and/or less than or equal to 10, in particular less than or equal to 8.

The fastening element can comprise lines of hooks and columns of hooks (projections), the hook line being perpendicular to the hook column. The hooks of one line can comprise a same shape of hooks and/or the hooks of one column can comprise a same shape of hooks. The hooks of one line can be orientated in the same direction and/or the hooks of one column can be orientated in the same direction. The hooks of one line can be alternately in opposite directions and/or the hooks of one column can be alternately in opposite directions. The alternation of hooks in opposite direction in one line and/or column is so called "dual hook" configuration. In "dual hook" configuration, hooks can be spaced each other or grouped by pair of two adjacent hooks such that two adjacent hooks are joined facing a pair of hook-shaped injection-molded projections (i.e. by a part of a lateral surface) and only pairs of hooks are spaced apart. The effect of the present invention can be more expressed for the fastening elements arranged in "dual hook" configuration and/or in pair configuration where the demolding can affect the shape of some hooks than others, especially depending of the position of the axe of demolding in comparison with the orientation of the hook cavity.

The thickness of the base 1 may be between one of lower limits of 0.05 mm, in particular 0.1 mm, preferably 0.2 mm and one of upper limits of 6 mm, in particular 5 mm, preferably 4 mm. The present invention finds a special application for such thin base thicknesses where such bases are difficult to manufacture, especially in injection-molding, and thus the effect of the present invention can be more expressed for base thickness in these ranges.

In a view onto the surface of the base 1 of the fastening element on which the retaining elements are formed, two longitudinal edges and two transversal edges may be present, wherein
- the maximum distance between the longitudinal edges and perpendicular to at least one of the longitudinal edges is defined as a maximal width of the fastening element,
- the maximum distance between the transversal edges and perpendicular to at least one of the transversal edges is defined the maximum length of the fastening element,
and the ratio of the maximal width to the maximal length may be between one of lower limits of 0.1, in particular 0.15, preferably 0.20, more preferably 0.25 or 0.30 and one of upper limits of 0.5, in particular 0.45, preferably 0.40. The present invention finds a special application for such asymmetrical dimension of the base that are deformation-sensitive and thus difficult to manufacture, especially for injection-molding, and where the effect of the present invention can be more expressed for base dimensions in these ranges.

The base 1 of a fastening element may comprise a thickness extending in a direction perpendicular to the surface of the base 1 on which the retaining elements are formed. In a view onto this surface of the base, the fastening element comprises two longitudinal edges and two transversal edges, wherein
- the maximum distance between the longitudinal edges and perpendicular to at least one of the longitudinal edges is defined as a maximum width (Wmax) of the fastening element,
- the maximum distance between the transversal edges and perpendicular to at least one of the transversal edges defined a maximum length (Lmax) of the fastening element,
and Lmax is higher than Wmax, and the ratio of the maximum length (Lmax) to the thickness of the base may be between one of lower limits of 10, in particular 15, preferably 20 and one of upper limits of 1500, in particular 1000, preferably 800, more preferably 600. The present invention finds a special application for such asymmetrical dimension of the base that are deformation-sensitive and thus difficult to manufacture especially for injection-molding, and where the effect of the present invention can be more expressed for base dimensions in these ranges.

The fastening element may have a total height measured as the maximum distance between two parallel main surfaces of the base 1 with a ratio of total height of the fastening element to the thickness of the support or the base 1 between one of lower limits of 1, in particular 1.1, preferably 2 and one of upper limits of 25, in particular 20, preferably 17. The effect of the present invention can be more expressed when the retaining elements are in these ranges.

The base 1 may comprise at least a rib and/or a gap extending between two adjacent rows of hooks and/or lines of hooks.

The fastening element may be for a vehicle, in particular for the fixation of a decorative element of the interior of the vehicle, such as automotive vehicle.

With hooks as the retaining elements, along a hook's height measured perpendicular to the surface of the base 1 on which it is formed, there may be arranged a "thinning" above or equal to the 1/10 of the height of the hook, in particular above or equal to 2/10 of the height of the hook, and/or below the 9/10 of the height of the hook, in particular below the 8/10 of the height of the hook. The hook may also comprise two "thinnings", each arranged on two opposite sides of the hook. With such "thinning", the hooks have a better capacity to retain counterpart comprising loops, such loops having less capacity to slide out of the "thinning" (see namely Figures 2, 4A and 4B).

The fastening element of the invention may exert its functions and may be used in the following contexts.

A vehicle comprises a chassis, and the fastening element is fixed directly or indirectly to the chassis.

A vehicle comprises a decorative element comprising retaining elements arranged to cooperate with a plurality of hooks of the fastening element such that the decorative element may fully cover and hide the fastening element.

A decorative element is part of the interior of the vehicle. The decorative element is at least one of a door panel, a dashboard panel, a door-side armrest, a glovebox, a door-side glovebox, a roof panel (such as sunroof), a trunk panel, a headliner, a floor panel, a load floor panel, cargo management structure, automotive accessories, a carpeting.

### Material options for the fastening element

For the support or base 1 and/or the retaining elements of the fastening element suitable resin raw materials may be selected from one or more of the following raw materials:
- Acrylonitrile butadiene styrene (ABS), acrylonitrile styrene acrylate (ASA), polystyrene (PS), styrene acrylonitrile copolymer (SAN), styrene methyl methacrylate (SMMA), styrene butadiene copolymer (SBC), SPS, high impact polystyrene (HIPS), general purpose polystyrene (GPPS), methyl methacrylate butadiene styrene (MMBS), methacrylate acrylonitrile butadiene styrene (MABS),
- Polyamide (PA) or Nylon type, comprising aliphatic polyamides homopolymers such as polyamide 6 (PA6), polyamide 6.6 (PA66), polyamide 12 (PA12), polyamide 11 (PA11), PA6.10, PA 6.12, PA10.10, PA 4.6, and/or its copolymers such as PA66/6, PA6/6.6/10, and/or polyphthalamides such as PA6I, PA6T, PA66T, PA9T, PAMXD 6, and/or aramides (aromatic polyamides).
- Polyethylene (PE) type (being a polyolefin), such as high density polyethylene (HDPE), low density polyethylene (LDPE), linear low density polyethylene (LLDPE), ultra high molecular weight polyethylene (UHMWPE), medium density polyethylene (MDPE), chlorinated polyethylene (CPE),
- Polycarbonate (PC), polyimide (PI), poly(methyl methacrylate) (PMMA), polyoxymethylene (POM), Poly(phenylene oxide) (PPO), polyether block amide (PEBA), polyether ether ketone (PEEK), polyethylenimine (PEI), polyphenylene sulfide (PPS), polysulfone (PESU), polyphenylsulfone (PPSU), polyphtalamide (PPA), polyvinylidene fluoride (PVDF), polypropylene carbonate (PPC), polyphenyl ether (PPE), polyketone (PK), polyetherketoneketone (PEKK), polyaryletherketone (PAEK), perfluoroalkoxy alkanes (PFA), polyethylene terephthalate (PET), polyethylene terephthalate glycol (PETG), polybutylene terephthalate (PBT), polycyclohexylene dimethylene terephthalate (PCT), poly cyclohexylenedimethylene terephthalate glycol-modified (PCTG), polycyclohexylenedimethylene terephthalate acid (PCTA), polypropylene (PP) being a polyolefin, polyamide-imide (PAI), ethylene-chlorotrifluoroethylene (ECTFE), polytetrafluoroethylene (PTFE), ethylene tetrafluoroethylene (ETFE), fluorinated ethylene propylene (FEP), cellulose acetate (CA), cellulose acetate butyrate (CAB), cyclic olefin copolymer (COC), cellulose propionate (CP), poly(ethylene-co-methacrylic acid (E/MAA), ethylene vinyl acetate (EVA), liquid crystal polymers (LCP), polyvinyl chloride (PVC), polylactic acid (PLA), polybutylene adipate terephthalate (PBAT), polybutylene succinate (PBS), thermoplastic elastomers (TPE), thermoplastic polyurethane (TPU), thermoplastic vulcanizate (TPV), plastomer (POE), thermoplastic polyester elastomer (TPEE, TPC), thermoplastic olefin (TPO),
- or a combination using one or more of the above-mentioned raw materials, in particular a combination using at least two raw materials such as ABS/PA6, ABS/PBT, ABS/PET, PC/ABS, PC/PBT, PC/PET, PSU/PC, PVC/NBR, PVC/PUR, LDPE/LLDPE, and/or a combination of three raw materials such as PC/ABS/SMA, PC/ABS/PMMA.

For the support or base 1 and/or the retaining elements of the fastening element resin raw materials may be selected from amorphous resins such as ABS, ASA, AS, PS, SAN, SMMA, SBC, HIPS, GPPS, MMBS, PC, PPE, PMMA, PPO, PEI, PPA, PPC, PPE, PESU, PET, PETG, CA, CAB, PVC, TPU, and POE. In particular suitable amorphous thermoplastic resins are those disclosed in US 2015/0313320 A1 here incorporated by reference.

For the support or base 1 and/or the retaining elements of the fastening element resin raw materials may be selected from crystalline or semi-crystalline resins are e.g. MABS, PA6, PA66, PA66T, PA 6.12, PA11, PA12, PA66/6, PEBA, HPPA, PA9T, PA6I, PA6T, PA61/6T, PA612, PA610/PA66, PA610, PA6/6T, PA6/69, PA6/3T, PA6/12, PA46, PA1010, Nylon, HDPE, LDPE, LLDPE, UHMWPE, mMDPE, MDPE, , LMDPE, mLLDPE, CPE, PI, POM, PEEK, PPS, PPSU, PVDF, PK, PI, PEKK, PAEK, PFA, PBT, PP, PAI, PAMXD 6, ECTFE, PTFE, FEP, COC, CP, E/MAA or IO, E/TFE, EVA, LCP, PLA, PBAT, PBS, TPE, TPV, TPEE, TPC and TPO.

For the support or base 1 and/or the retaining elements of the fastening element resin raw materials may be selected from a combination of at least one amorphous resin and/or at least one crystalline resin and/or at least one semi-crystalline resin.

In some embodiment, the raw material can comprise glass fibers.

Exemplary annealing temperatures for such materials are e.g. 90±5°C for ABS, 150±5°C for PC/ABS or 170±5°C for PC, e.g. for 20 to 30 seconds with an adapted distance as mentioned later in the description. Annealing temperatures and times are chosen to transfer a solid state of an as molded article into a deforming state without excessive deformation and without transfer into a melt. A particular material preference is PC/ABS. According to a further preference the material may be provided with a heat absorbing, in particular an IR absorbing color, e.g. with a carbon black additive.

A base side portion and the retaining elements like hooks may be made from at least one crystalline material or are preferably made from an amorphous or substantially amorphous thermoplastic resin, wherein an amorphous material means a material of which the level of crystallinity within the material is 0% and a substantially amorphous means a material containing amorphous zones and non-amorphous zones, the amorphous zones representing more than half of the volume of the material in question. Preferably, its represent more than 60%, more preferably still, more than 70%, very preferably, more than 80%, and preferably more than 95% in terms of volume.

The base side portion may be one which in case of hooks as retaining elements has a bending modulus as indicated in US 2015/0313320 A1 here incorporated by reference like at least 1000 MPa, in particular higher than 1200 MPa, preferably higher than 1500 MPa, or even 2000 MPa and/or up to 5000 MPa, preferably 3500 MPa or even 3000 MPa.

The support and/or base 1 may be fixed by overmolding at least a part of it. The support and/or base 1 and the retaining elements like hooks and may be formed integrally of a same raw material or of di▪ erent raw materials for the support or base 1 and the hooks or of di▪ erent raw materials of the same kind for the support or base 1 and the hooks. The retaining elements like hooks may be formed of a single material.

### Method for producing a fastening element

A method of producing at least a portion of a fastening element having a surface and retaining elements projecting from the surface, comprises
- injection-molding into cavities of at least a part of a mold,
- imparting conversion to structures, obtained in said cavities from said injection-molding, into deformed structures by demolding.

According to the invention annealing is performed for reversing (at least partially, preferably at least 20%, notably 30% of the deformed structures) an extent of said conversion by application of energy to at least parts of the deformed structures in a released state of being exposed to an atmosphere.

This method of producing according to the invention may be a method of producing at least a portion of the or the entire fastening element of the invention.

The method allows in particular forming a plurality of hooks arranged to cooperate with a counterpart made of hooks and/or of loops for making a gripping fastener attachment, each of the hooks comprising a stem part connected to the base 1 and a head part or arc-shaped portion arranged at the top of the stem part, the head part of the hooks having a convex upper side and/or a concave lower side and/or a flat upper side.

The annealing process reduces the internal residual constraints/stress of the structures to be subjected to annealing like intermediate hooks of the fastening element. The intermediate hook comprises preferably a stem part and an anchoring part extending laterally of the stem part. The anchoring part is more or less pronounced depending on the used raw material and in some cases the anchoring part extend in the prolongation of the stem part.

A source of energy for the annealing may be arranged on the side of the surface of the base 1 on which the retaining elements are arranged. The annealing is made by providing energy to at least parts of the intermediate hooks using a fluid but no tool which contacts retaining elements (such as a roll). The fluid may not be particularly supplied but can be the natural atmosphere and is preferably not pressurized. For the annealing suitable energy sources are at least one of infrared radiation, hot air, hot liquid and steam. Convection or heat conduction means or a combination thereof may also be used. Impinging a heated fluid such as hot water or equivalent could be also used.

The annealing process reduces the height of the intermediate hooks by at least 5%, in particular 10%, preferably 15%, and/or up to 70%, in particular 55%, preferably 45% so as to achieve the height of the (final) hooks as retaining elements of the fastening element. This height reduction is preferably an average height reduction for hooks on the fastening element and may also apply to fastening elements with other retaining elements than hooks. By varying the temperature and/or the time of exposure and/or number of annealing applications and/or the type of energy source(s), the height reduction will be more or less significant and the obtained annealed retaining elements will be more or less functional. Thus, the effect of the invention will be more or less expressed.

In absolute values, the height of the intermediate hooks may be reduced from at least 0,05 mm, in particular 0,1 mm, preferably 0,2 mm, and/or up to 0,7 mm, in particular 0,55 mm, preferably 0,45 mm.

The fastening element may be an article which is a single piece, obtained by injection-molding at least one injection point and it can be obtained in a single piece via injection-molding at one or more injection points with the invention's method. The molded article may comprise on its outer surface at least one area in the form of a hollow or point, corresponding to a point of injection of thermoplastic resin during the injection-molding.

To understand the invention's concept,
- Figure 2 presents a microscopic view on a pair of hook-shaped injection-molded projections on a base from a crystalline thermoplastic resin,
- Figure 4B presents a microscopic view on a pair of hook-shaped injection-molded projections on a base from an amorphous thermoplastic resin obtained according to the invention by annealing the intermediate product shown in Figure 4A. The cavity shapes are the same for hooks of Figures 2 and 4A.

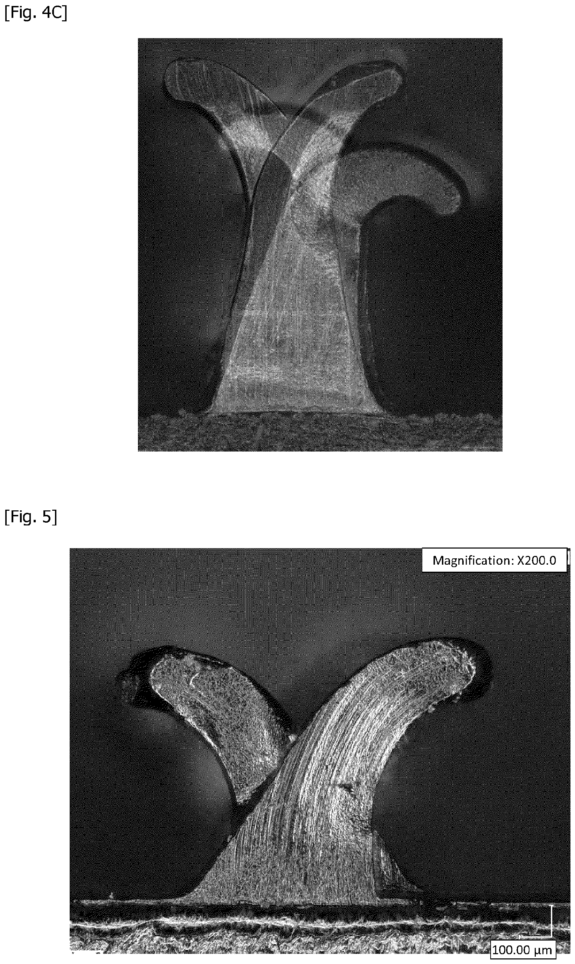

In contrast to the case shown in Figure 2 and the use of a crystalline thermoplastic resin, the use of an amorphous (thermoplastic) resin in injection-molding imparts by demolding a significant extend of conversion to an injection-molded structure as it is apparent from a comparison of Figure 4A with Figure 4B. For Figures 4A and 4B, annealing serves to reverse an extent of the conversion as it is apparent from Figure 4C that is the superposition of Figures 4A and 4B. In this sense the conversion is a deformation of an injection-molded structure established in the cavity of the mold.

### Surface measurements and observations on retaining elements

Samples were prepared from fastening elements produced according to a method of producing a portion of a fastening element having a surface and hooks as retaining elements projecting from the surface, comprising
- injection-molding into cavities of at least a part of a mold,
- imparting conversion to structures, obtained in said cavities from said injection-molding, into deformed structures by demolding.

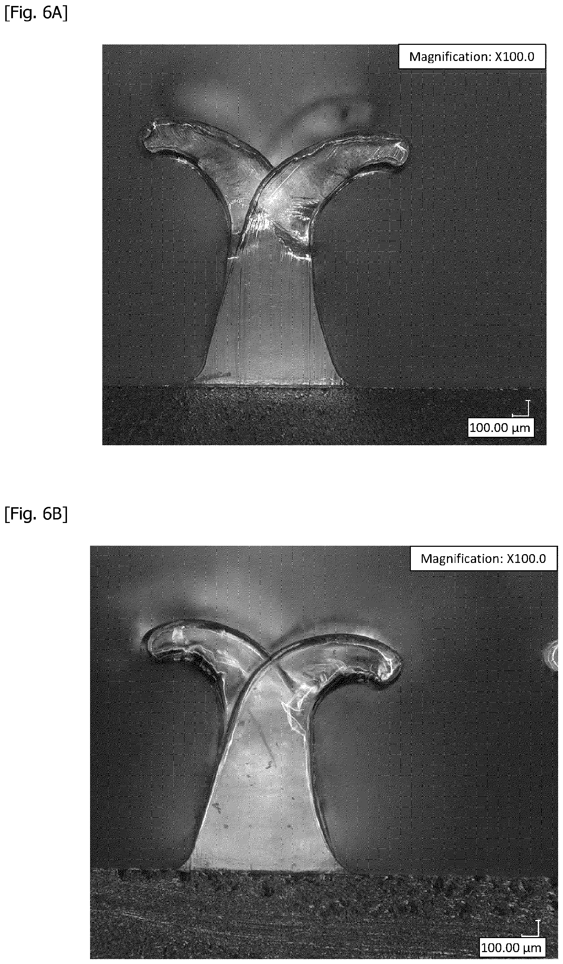

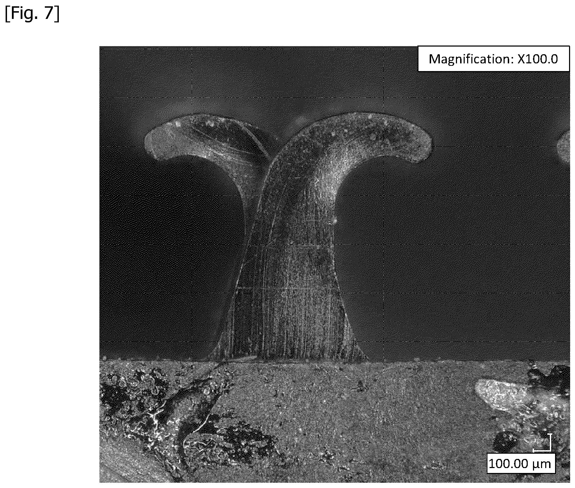

The fastening elements produced were in the shape of cards on a surface of which an array of hooks or pairs of hooks was formed. The cards were cut only in an area void of hooks, along a row of hooks, for example with scissors, paying attention not to damage the hooks. Microscopic views at an adapted magnification depending of the hook dimensions are shown in Figures 1 to 7. Measurement standards observed for specific aspects of obtaining surface profile parameter values were, for example, ISO 21920 and ISO 13565 for surface profile parameters and ISO 25178 for texture parameters.

Values of Str, Ssk and Sku are values from measurements in accordance with ISO 25178-2:2021 and the surface measurements and observations on the cuts are obtained with the measurement system available under the trademark Alicona G4 InfiniteFocus from Alicona Imaging GmbH, Dr. Auner Straße 19, 8074 Raaba/Graz, Austria. The measurement system allows measuring form and roughness of surfaces. It combines the small depth of focus of an optical system with vertical scanning to provide topographical and color information from a variation of focus. A cuto▪ of 2.5pm for λs and a cuto▪ of 0.08 mm for λc and rectangular measurement surface areas of at least 0.02 mm² are used to obtain the values of Str, Ssk and Sku. Textural or surface profile di▪ erences are measured outside of isolated asperities of an observed surface.

Specifically,
- Str, the Texture Aspect Ratio, is a measure of the spatial isotropy or directionality of a surface (texture) and for a surface with regular profile Str parameter will tend towards 0.00, whereas a spatially isotropic texture/profile will result in a Str of 1.00,
- Ssk values represent the degree of bias of the roughness shape (asperity) with a height distribution skewed above the mean plane resulting in Ssk<0, a height distribution (peaks and pits) symmetrical around the mean plane resulting in Ssk=0, and a height distribution skewed below the mean plane resulting in Ssk>0, and
- Sku value is a measure of the sharpness of the roughness profile with a height distribution skewed above the mean plane resulting in Sku<3, a height distribution which is normal (sharp portions and indented portions co-existing) resulting in Sku=3, and a height distribution spiked resulting in Sku>3.

The invention provides fastening elements as follows:
A fastening element having a base surface and injection-molded retaining elements at least partially made of an amorphous resin and/or crystalline resin and projecting from the base surface up to and with a retention portion, wherein a further surface being a surface of each of the retaining elements and visible in a view showing the retention portion and onto a plane, on which the retaining element extends between the retention portion and the base surface, has an area comprising a profiled area, wherein, when subjected to an analysis according to the description of the retaining elements, said profiled area shows, when measuring in accordance with ISO 25178-2:2021, at least one of
a) spatial isotropy or directionality of a texture with a value of Str above 0.20 and/or up to 1.0,
b) symmetry with regard to a mean line of a roughness profile with a value of Ssk above 2.3 or below -1, and
c) sharpness of a roughness profile with a value of Sku above 5 or below 2.

The fastening element according to the invention, wherein the profiled area is proximal to the base surface and said area of the further surface further comprises an area (3) distal to the base surface, and when analyzed in a same manner, said proximal and said distal area differ from each other in terms of at least one of
a) the spatial isotropy or directionality of a texture,
b) the symmetry with regard to a mean line of a roughness profile, and
c) the sharpness of a roughness profile.

The fastening element according to the invention, wherein in a visual microscopic observation of the further surface from the proximal to the distal area grooves (2) observed in the proximal area disappear in the distal area (3).

The fastening element according to the invention, wherein a ratio of at least one of said values to a value for the corresponding parameter of the distal area (3) is 1.1 or more.

The fastening element according to the invention, wherein the retaining portion projects from the base surface up to and with a curvature at an apex.

The fastening element according to the invention, the retaining elements of which are hooks.

The fastening element according to the invention, wherein the profiled area shows said value of Str above 0.20 and/or up to 1.0.

The fastening element according to the invention, wherein said value of Str is above 0.20 and not more than 1.0 with a cutoff as defined in the description of 2.5 µm for λs and 0.08 mm for λc.

The fastening element according to the invention, wherein said value of Str is between 0.25 and 1.0.

The profiled area is proximal to the base surface and/or distal the base surface, preferably the profiled area is only distal to the base surface.

The one or more of the differences are preferably observed in square area sections of 0.02 mm² or preferably 0.1 mm².

Figure 1 shows that in a visual microscopic observation of the further surface from the proximal to the distal area grooves observed in the proximal area disappear in the distal area.

In an average of at least 2 or preferably 3 of the retaining elements, said areas differ from each other with a value ratio above 1 having an absolute value for the proximal area as a divisor and from values for one of
a) spatial isotropy or directionality of a texture,
b) symmetry with regard to a mean line of a roughness profile, and
c) sharpness of a roughness profile.

This ratio is in particular 1.1 or more, preferably 1.3 or more and the numerator of this ratio is one of
a) a value of Str above 0.2,
b) a value of Ssk above 2.3 or below -1, and
c) a value of Sku above 5 or below 2.

Preferably this value of Str is within a range having one of the lower limits of 0.25, preferably 0.35 and more preferably 0.55 and/or one of the upper limits of 1.0, in particular 0.95, preferably 0.90, more preferably 0.85, particularly preferably 0.80.

Observing these limits allows advantages of:
- having a retention higher than that obtained directly after demolding,
- having reduced residual stress and having a geometrical shape closest to the molding cavity shape and su▪ cient gripping performances,
- having a surface aspect with a low orientation or without orientation allowing the counterpart to slide better on this surface to access to the retention cavity,
- obtaining grip performance in consistent/homogeneous manner as the material is shaped homogeneously,
- similar mechanical properties of the distal area of the hook in roughly all directions,
- having an isotropic surface of the hook, e.g. by erasing brittleness/weakness and/or the susceptibility to rupture due to the demolding,
- a crust, in particular a homogeneous crust in an area, where an arc shape of the hook is formed,
- in this area where an arc shape of the hook is formed, forming the surface of the hook with an improved skin, for example a Goosebumps skin or bulk (pellet) raw material aspect or sand aspect.

A hook as a retaining element comprising a first surface finish having a first value of Str, Str1 value, and a second surface finish having a second value of Str, Str2 value, wherein Str1 is below the Str2, and wherein one of the Str1 value and Str2 value is above 0,25, and the ratio of the Str2 and Str1 is between 0,7 and 50, in particular, between 1,0 and 40, some cases between 2,5 and 30, for example Str2 is arranged at an area closed to the head part and the Str1 is arranged at an area of the stem part in particular an area of the stem part that is close to the base surface, or the opposite.

Preferably, the value of Ssk is within a first range and/or a second range, the first range having one of the lower limits of 1, in particular 2, more preferably 3 and/or one upper limits of 45, in particular 20, more preferably 10, the second range having one of the lower limits of -45, in particular -20, more preferably -12 and/or one upper limits of 0, in particular -1, more preferably -2. A hook as a retaining element comprising a first surface finish having a first value of Ssk, Ssk1 value and a second surface finish having a second value of Ssk, Ssk2 value, wherein Ssk1 is below the absolute of Ssk2 and the ratio of the absolute Ssk2 and absolute Ssk1 is between 2 and 60, in particular, between 2,5 and 20, in some case, between 3 and 15, for example Ssk2 is arranged at an area closed to the head part and the Ssk1 is arranged at an area of the stem part in particular an area of the stem part that is close to the base surface, or the opposite.

The Ssk parameter is, as the Str and the Sku, representative for surface features of the state of the hook and reflects the low residual stress of the hook. With preferable ranges of these parameters according to the invention, the hook has a geometric shape as close as possible to the shape of the molding cavity and the hook has su▪ cient grip performances with a loop counterpart.

Preferably this value of Sku is within a first range and/or a second range, the first range having one of the lower limits of 5, in particular 6, more preferably 7 and/or one upper limits of 150, in particular 100, more preferably 80. A hook as a retaining element comprising a first surface finish having a first value of Sku, Sku1 value and a second surface finish having a second value of Sku, Sku2 value, wherein Sku1 is below the absolute of Sku2 and the ratio of the absolute Sku2 and absolute Sku1 is between 2 and 30, in particular, between 3 and 20, for example Sku2 is arranged at an area closed to the head part and the Sku1 is arranged at an area of the stem part in particular an area of the stem part that is close to the base surface, or the opposite.

Figure 1 presents a microscopic view (here a photography) with 100-fold magnification onto a portion of a fastening element according to the invention, specifically onto a cut in accordance with the above-described sample preparation, with a hook-shaped injection-molded projection of thermoplastic amorphous resin, specifically PC/ABS. In other words and more generally, a first zone and the second zone have a textural di▪ erence and/or a surface aspect di▪ erence. Such di▪ erence is obtained and is a consequence of the annealing of the hook head in producing the fastening element according to the invention. The purpose of the annealing is to reduce the constraint/strain resulting from demolding step of the resin or material forming the intermediate hooks (structures obtained after demolding).

As visible in Figure 1 a first area or zone comprises longitudinal grooves 2 and a second zone or area 3 is free from longitudinal grooves. Such longitudinal grooves may extend in a direction perpendicular to the plane of the base 1 and/or in a direction inclined at ± 45° of the direction perpendicular to the first surface of the base, in particular in a direction inclined at ± 30° of the direction perpendicular to the first surface of the base. Such longitudinal grooves may be continuous longitudinal grooves, in particular continuous longitudinal grooves being continuous over a length of one eighth of the height of the hook, in particular a fifth, a quarter, a third of the height of the hook.

The hook visible in Figure 1 comprises a stem part connected to the base and a head part arranged at the top of the stem part, the first part is a section of the stem part, and the second part is a section of the head part. The hook comprises a portion with a trapezoid contour and an arc-shaped portion. A lateral view of the hook shows an external curvature of the hook with an inflexion point.

As described above along a hook's height measured perpendicular to the surface of the base 1 on which it is formed, there may be arranged a "thinning" above or equal to the 1/10 of the height of the hook, in particular above or equal to 2/10 of the height of the hook, and/or below the 9/10 of the height of the hook, in particular below the 8/10 of the height of the hook. The hook may also comprise two "thinnings", each arranged on two opposite sides of the hook, in particular its stem part.

A "Hook area" may be understood to be the area delimited by a line which is closed, passing via the perpendicular direction such that all perpendicular projections of hook stems are situated on the line or inside it. Preferable lower limits for the hook area are 30%, more preferably 40%, particularly preferably 45% or 50% or even 55% or 60% or most preferably 65% or 70% of an observed hook surface. The fastening element comprises a support or base 1 which is continuously straight or curved. The perpendicular direction is perpendicular to a tangent or to the continuously straight surface of the support or base 1 on which the hooks are formed.

### Specific investigated samples

As an example, material and processing specifications may be used for a production and a product according to the invention as listed in Table 2.

**[Table 2]**

| | |
|---|---|
| cycle time | classical and depending on the volume / shape of the product to be molded |
| mold | conventional metallic mold |
| annealing temperature, time | 150±5°C, 3±2 s |
| annealing heat source | 4 modules of a twin-tube carbon infrared emitter each having a power of 65±10 kW/m² and a wavelength of 2± 1 µm have been placed above and essentially parallel to a surface of the base of the molded article in a non-pressurized atmosphere (i.e. normal or natural atmosphere) to establish the annealing temperature at a distance of 100±20 mm from the protrusion (non-annealed hook) of the molded article towards the heat source. |

The samples of the material PC/ABS below were prepared with theses specifications.

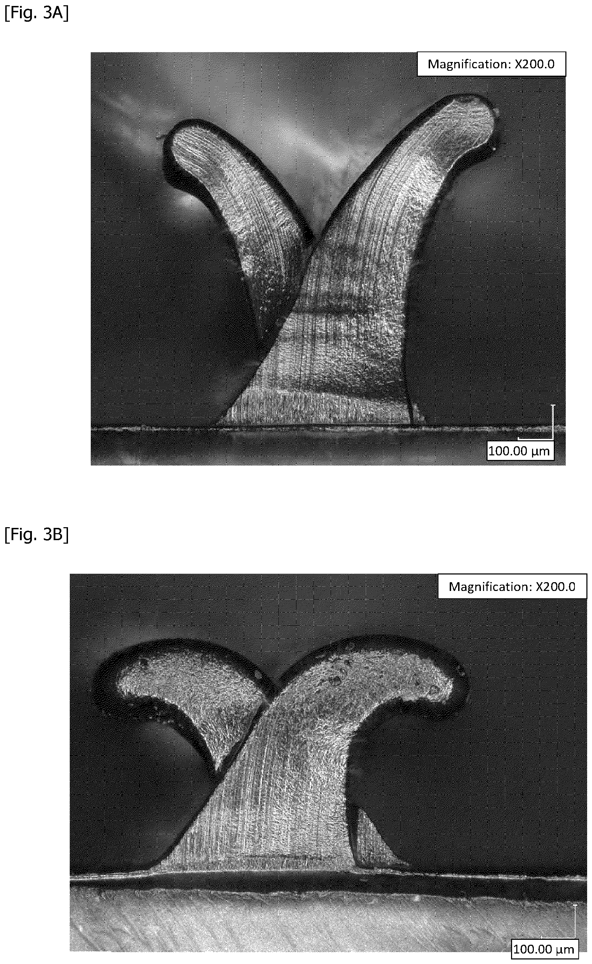

In line with the above section 'Surface measurements and observations on retaining elements', samples were prepared via single piece-injection-molding and imaged with an adapted fold magnification lens depending of the dimensions of the hooks, for example a 100-fold magnification lens or 200-fold magnification lens as made for the images being shown in Figures 3A to 7 using a microscope from KEYENCE designated under the reference VHX-6000. Samples as shown in Figure 3A, Figure 4A and Figure 6A were used as intermediate products to be converted into samples shown in Figure 3B, Figure 4B and Figure 6B, respectively, via annealing. The samples, related figures and their annealed or non-annealed (e.g. intermediate product) character are indicated in the Table 3:

**[Table 3]**

| sample label | material | annealed sample | image |
|---|---|---|---|
| Aplix' Mini Hook | PC/ABS (amorphous resin) | no (as molded) | Figure 3A |
| Aplix' Mini Hook | PC/ABS (amorphous resin) | yes (after annealing) | Figure 3B |
| Aplix' Dual Hook | PC/ABS (amorphous resin) | no (as molded) | Figure 4A |
| Aplix' Dual Hook | PC/ABS (amorphous resin) | yes (after annealing) | Figure 4B |
| Aplix' Mini Hook | PA66 (crystalline resin) | no (as molded) | Figure 5 |
| Aplix' Dual Hook | PC (amorphous resin) | no (as molded) | Figure 6A |
| Aplix' Dual Hook | PC (amorphous resin) | yes (after annealing) | Figure 6B |
| Aplix' Dual Hook | PP (semi-crystalline resin) | no (as molded) | Figure 7 |

The label samples "Aplix' Mini hook" and "Aplix' Dual Hook" are well known Aplix' designations of different cavity molds. The "Aplix' Mini hook" being a dual hook as defined above with pair of joined hooks and with features corresponding to fastening type 3. The "Aplix' Dual Hook" being a dual hook as defined above with pair of spaced hooks and with features corresponding to fastening type 1.

Averages of observations of three hooks of each sample, specifically the entirely non hidden hooks in observations of the samples visible in Figures 3A to 4B were obtained as shown in Table 4.

**[Table 4]**

| | | | | |
|---|---|---|---|---|
| sample label | height of the front side structure | height of the front side structure | difference | variation |
| Aplix' Mini Hook | 1.040 mm in sample visible in Figure 3A | 0.701 mm in sample visible in Figure 3B | 0.313 mm | -32.60% |
| Aplix' Dual Hook | 1.904 mm in sample visible in Figure 4A | 1.591 mm in sample visible in Figure 4B | 0.313 mm | -16.43% |

Samples, parts of which are shown in above-mentioned figures (indicated as respective related figures below), were subjected to the above-described measurements in accordance with ISO 25178-2:2021 in base-proximal and base-distal areas as visible in these figures to obtain the results shown in Table 5 and subjected to mechanical property measurements before and after indicated cycles of attaching and detaching the samples to obtain the results shown in Table 6. In Table 5, Str1, Ssk1 and Sku1 are values from the proximal areas and Str2, Ssk2 and Sku2 are values from the distal areas.

**[Table 5]**

| **Fastening type** | **Zone of the hook sample** | **Material** | **HOOK sample** | **Location of the measured hook on the part** | **Surface measured (mm²)** | **Cut off λS (µm)** | **Cut off λC (mm)** | **Str** | **Ssk** | **Sku** | ratio Str (Str2/Str1) | ratio Ssk (ABS(Ssk2)/ABS(Ssk1)) | ratio Sku (Sku2/Sku1) |
|---|---|---|---|---|---|---|---|---|---|---|---|---|---|
| 1 as molded | Proximal | PC/ABS | 1 | center | 0,0459 | 2,5 | 0,08 | 0,1 | 1,4 | - | 2,00 | 1,43 | - |
| | Distal | PC/ABS | 1 | center | 0,0236 | 2,5 | 0,08 | 0,2 | 2 | - | | | |
| 1 as annealed | Proximal | PC/ABS | 2 | center | 0,0517 | 2,5 | 0,08 | 0,03 | 0,62 | 3,3 | 24,00 | 9,84 | 20,88 |
| | Distal | PC/ABS | 2 | center | 0,0443 | 2,5 | 0,08 | 0,72 | 6,1 | 68,9 | | | |
| 1 as annealed | Proximal | PC/ABS | 3 | center | 0,0517 | 2,5 | 0,08 | 0,06 | 0,3 | 4 | 10,17 | 9,00 | 4,95 |
| | Distal | PC/ABS | 3 | center | 0,0627 | 2,5 | 0,08 | 0,61 | 2,7 | 19,8 | | | |
| 1 as annealed | Proximal | PC/ABS | 4 | side | 0,0517 | 2,5 | 0,08 | 0,45 | -1,2 | 25,6 | 1,27 | 0,67 | 0,12 |
| | Distal | PC/ABS | 4 | side | 0,0443 | 2,5 | 0,08 | 0,57 | 0,8 | 3 | | | |
| 3 as annealed | Proximal | PC/ABS | 5 | center | 0,0342 | 2,5 | 0,08 | 0,44 | -1,8 | 128 | 1,57 | 3,06 | 0,60 |
| | Distal | PC/ABS | 5 | center | 0,0251 | 2,5 | 0,08 | 0,69 | 5,5 | 77 | | | |
| 3 as annealed | Proximal | PC/ABS | 6 | center | 0,0211 | 2,5 | 0,08 | 0,63 | 0,1 | 7,1 | 1,32 | 52,00 | 8,17 |
| | Distal | PC/ABS | 6 | center | 0,0211 | 2,5 | 0,08 | 0,83 | 5,2 | 58 | | | |
| 3 as annealed | Proximal | PC/ABS | 7 | side | 0,0211 | 2,5 | 0,08 | 0,07 | 0,7 | 7,6 | 12,43 | 7,00 | 4,08 |
| | Distal | PC/ABS | 7 | side | 0,0211 | 2,5 | 0,08 | 0,87 | 4,9 | 31 | | | |
| 1 as annealed | Proximal | PC | 8 | side | 0,0211 | 2,5 | 0,08 | 0,5 | -0,3 | 13 | 1,54 | 6,33 | 1,31 |
| | Distal | PC | 8 | side | 0,0211 | 2,5 | 0,08 | 0,77 | 1,9 | 17 | | | |
| 1 as annealed | Proximal | PC | 9 | side | 0,0211 | 2,5 | 0,08 | 0,77 | -8,6 | 861 | 0,92 | 0,44 | 0,03 |
| | Distal | PC | 9 | side | 0,0211 | 2,5 | 0,08 | 0,71 | 3,8 | 29 | | | |
| 1 as annealed | Proximal | PC | 10 | side | 0,0211 | 2,5 | 0,08 | 0,74 | 2 | 24 | 1,05 | 2,75 | 2,21 |
| | Distal | PC | 10 | side | 0,0618 | 2,5 | 0,08 | 0,78 | 5,5 | 53 | | | |

Hook samples in Table 5 correspond to different hooks of a same or different hook area(s) of a same or different article and/or card.

**[Table 6]**

| Fastening element type 1 | | | PC/ABS | | | PC | | |
|---|---|---|---|---|---|---|---|---|
| | | | As-molded | Annealed | Comparison between as-molded and annealed stage; Improvement due to annealing | As-molded | Annealed | Comparison between as-molded and annealed stage; Improvement due to annealing |
| Gripping properties | Shear strength (ASTM D5169) | psi | 51,7 | 81,9 | 58% | 39,95 | 77,61 | 94% |
| | Peel strength (ASTM D5170 AX) | lbs/in | 5,1 | 9,6 | 88% | 3,71 | 11,37 | 206% |
| | Peel strength after 30 cycles (ASTM D5170 AX) | lbs/in | 3,7 | 5,4 | 46% | 3,14 | 5,86 | 87% |
| | Tensile strength | psi | 15,5 | 40,9 | 164% | 13 | 40,42 | 211% |
| | Tensile strength after 5 cycles | psi | 15,7 | 34,7 | 121% | 14,91 | 36,53 | 145% |
| Related figure | | | 4A | 4B | | 6A | 6B | |

Although the present invention has been described with reference to specific exemplary embodiments, it is obvious that modifications and changes can be carried out on these examples with departing from the general scope of the invention as defined by the claims. In particular, individual features of the different embodiments illustrated/mentioned can be combined into additional embodiments. Consequently, the description and the drawings must be considered in an illustrative, rather than a restrictive sense.

It is also obvious that all the features described with reference to a method are transposable, alone or in combination, to a device, and conversely, all the features described with reference to a device are transposable, alone or in combination, to a method.

## Claims

1. A method of producing at least a portion of a fastening element having a surface and retaining elements projecting from the surface, comprising
- injection-molding into cavities of at least a part of a mold,
- imparting conversion to structures, obtained in said cavities from said injection-molding, into deformed structures by demolding,
**characterized by**
- annealing for reversing an extent of said conversion by application of energy to at least parts of the deformed structures in a state of being fully exposed to a fluid.

2. The method according to claim 1, wherein said energy is heat energy and/or infrared energy.

3. The method according to claim 1 or 2, wherein said injection-molding into cavities is performed with an amorphous resin.

4. The method according to claim 1 or 2, wherein said injection-molding into cavities is performed with amorphous and non-amorphous resins to provide in each of the cavities a volume ratio of amorphous zones of resin and non-amorphous zones of resin of more than 1.

5. The method according to claim 4, wherein each of the structures obtained in the respective one of the cavities is composed of a resin comprising amorphous zones and non-amorphous zones, the amorphous zones represent more than 60% of the volume of the cavity, the remaining volume of the cavity being non-amorphous zones.

6. The method according to any one of claims 1 to 5, wherein on average the retaining elements are projecting from the surface to a height which is less than 90% of a height of projection from the surface of the deformed structures.

7. A fastening element having a base surface and injection-molded retaining elements at least partially made of an amorphous resin and/or crystalline resin and projecting from the base surface up to and with a retention portion, wherein
a further surface being a surface of each of the retaining elements and visible in a view showing the retention portion and onto a plane, on which the retaining element extends between the retention portion and the base surface, has an area comprising a profiled area, wherein, when subjected to an analysis according to the description of the retaining elements, said profiled area shows, when measuring in accordance with ISO 25178-2:2021, at least one of
a) spatial isotropy or directionality of a texture with a value of Str above 0.20 and/or up to 1.0,
b) symmetry with regard to a mean line of a roughness profile with a value of Ssk above 2.3 or below -1, and
c) sharpness of a roughness profile with a value of Sku above 5 or below 2.

8. The fastening element according to claim 7, wherein the profiled area is proximal to the base surface and said area of the further surface further comprises an area (3) distal to the base surface, and
when analyzed in a same manner, said proximal and said distal area differ from each other in terms of at least one of
a) the spatial isotropy or directionality of a texture,
b) the symmetry with regard to a mean line of a roughness profile, and
c) the sharpness of a roughness profile.

9. The fastening element according to claim 8, wherein in a visual microscopic observation of the further surface from the proximal to the distal area grooves (2) observed in the proximal area disappear in the distal area (3).

10. The fastening element according to claim 7, 8, or 9, wherein a ratio of at least one of said values to a value for the corresponding parameter of the distal area (3) is 1.1 or more.

11. The fastening element according to any one of claims 7 to 10, wherein the retaining portion projects from the base surface up to and with a curvature at an apex.

12. The fastening element according to any one of claims 7 to 11, the retaining elements of which are hooks.

13. The fastening element according to any one of claims 7 to 12, wherein the profiled area shows said value of Str above 0.20 and/or up to 1.0.

14. The fastening element according to claim 13, wherein said value of Str is above 0.20 and not more than 1.0 with a cutoff as defined in the description of 2.5 µm for λs and 0.08 mm for λc.

15. The fastening element according to claim 14, wherein said value of Str is between 0.25 and 1.0.
